(19) Europäisches Patentamt
European Patent Office
Office européen des brevets

(11) Veröffentlichungsnummer: **0 305 844 B1**

(12) **EUROPÄISCHE PATENTSCHRIFT**

(45) Veröffentlichungstag der Patentschrift: **23.09.92**

(51) Int. Cl.⁵: **C07D 249/12**, C07D 401/06, C07D 403/06, A01N 37/18, A01N 43/653

(21) Anmeldenummer: **88113611.3**

(22) Anmeldetag: **22.08.88**

Die Akte enthält technische Angaben, die nach dem Eingang der Anmeldung eingereicht wurden und die nicht in dieser Patentschrift enthalten sind.

(54) **Substituierte Triazolinone.**

(30) Priorität: **01.09.87 DE 3729070**

(43) Veröffentlichungstag der Anmeldung:
**08.03.89 Patentblatt 89/10**

(45) Bekanntmachung des Hinweises auf die Patenterteilung:
**23.09.92 Patentblatt 92/39**

(84) Benannte Vertragsstaaten:
**BE CH DE ES FR GB IT LI NL SE**

(56) Entgegenhaltungen:
**DE-A- 2 707 801**

**CHEMICAL ABSTRACTS, Band 87, Nr. 17, 24. Oktober 1977, Columbus, Ohio, USA; BANY, TADEUSZ et al.: "Derivatives of 1,2,4-triazole-5-thione. VI. Hydroxymethylation, chloromethylation, and formylation of 1,2,4-triazole-5-thione derivatives" Seite 713, Spalte 2, Zusammenfassung Nr. 135 170x**

**CHEMICAL ABSTRACTS, Band 89, Nr. 1, 13. Juli 1978, Columbus, Ohio, USA; RUDNICKA, WALERIA et al.: "Reaction of 1,2,4-triazole-3-thione with ethyl cloroformate" Seite 539, Spalte 2, Zusammenfassung Nr. 6 283d**

(73) Patentinhaber: **BAYER AG**

**W-5090 Leverkusen 1 Bayerwerk(DE)**

(72) Erfinder: **Findeisen, Kurt, Dr.**
**In der Follmühle 10**
**W-5068 Odenthal 2(DE)**
Erfinder: **Lindig, Markus, Dr.**
**Dahlienweg 16**
**W-4010 Hilden(DE)**
Erfinder: **Santel, Hans-Joachim, Dr.**
**Grünstrasse 9 a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Strang, Harry, Dr.**
**Unterdorfstrasse 6 a**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue substituierte Triazolinone, mehrere Verfahren zu ihrer Herstellung und ihre Verwendung als Herbizide.

Es ist bekannt, daß bestimmte Stickstoffheterocyclen wie beispielsweise das Imidazolidin-2-on-1-carbonsäureisobutylamid (vgl. z.B. K. H. Büchel "Pflanzenschutz und Schädlingsbekämpfung" S. 170, Thieme Verlag Stuttgart 1977) sowie Carbomoyl-1,2,4-triazole (EP-A-0 140 194) und Carbamoyl-1,2,4-triazolinone (JP-A-78 135 981) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolinone der allgemeinen Formel (I),

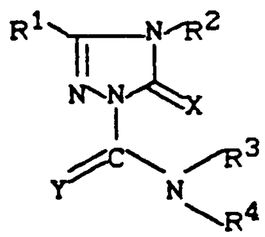

(I)

in welcher

| | |
|---|---|
| $R^1$ | für einen Rest |

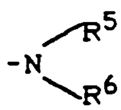

| | |
|---|---|
| | oder für einen Rest $-S(O)_n-R^7$ steht, wobei |
| $R^5$ und $R^6$ | unabhängig voneinander jeweils für Alkyl, Alkenyl, Alkinyl, Halogenalkyl, Halogenalkenyl, Halogenalkinyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind für einen gegebenenfalls substituierten Heterocyclus stehen, |
| $R^7$ | für Alkyl, Alkenyl, Alkinyl, Cycloalkyl, Cycloalkylalkyl oder für jeweils gegebenenfalls substituiertes Aralkyl oder Aryl steht und |
| n | für eine Zahl 0, 1 oder 2 steht, |
| $R^2$ | für Alkyl, Alkenyl, Alkinyl, Halogenalkyl, Halogenalkenyl, Halogenalkinyl, Alkoxyalkyl, Alkoxy, Cycloalkylalkyl, Cycloalkyl oder für jeweils gegebenenfalls substituiertes Aralkyl oder Aryl steht, |
| $R^3$ | für Wasserstoff oder Alkyl steht, |
| $R^4$ | für Alkoximinoalkyl steht, |
| X | für Sauerstoff oder Schwefel steht und |
| Y | für Sauerstoff oder Schwefel steht, |

gefunden.

Weiterhin wurde gefunden, daß man die neuen substituierten Triazolinone der allgemeinen Formel (I),

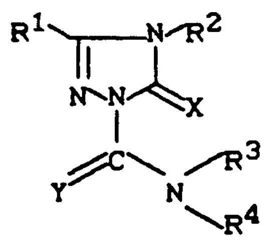

(I)

in welcher

$R^1$ für einen Rest

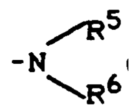

oder für einen Rest $-S(O)_n-R^7$ steht, wobei

$R^5$ und $R^6$ unabhängig voneinander jeweils für Alkyl, Alkenyl, Alkinyl, Halogenalkyl, Halogenalkenyl, Halogenalkinyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind für einen gegebenenfalls substituierten Heterocyclus stehen,

$R^7$ für Alkyl, Alkenyl, Alkinyl, Cycloalkyl, Cycloalkylalkyl oder für jeweils gegebenenfalls substituiertes Aralkyl oder Aryl steht und

n für eine Zahl 0, 1 oder 2 steht,

$R^2$ für Alkyl, Alkenyl, Alkinyl, Halogenalkyl, Halogenalkenyl, Halogenalkinyl, Alkoxyalkyl, Alkoxy, Cycloalkylalkyl, Cycloalkyl oder für jeweils gegebenenfalls substituiertes Aralkyl oder Aryl steht,

$R^3$ für Wasserstoff oder Alkyl steht,

$R^4$ für Alkoximinoalkyl steht,

X für Sauerstoff oder Schwefel steht und

Y für Sauerstoff oder Schwefel steht,

erhält, wenn man

a) 1-Chlor-(thio)-carbonyltriazolinone der Formel (II),

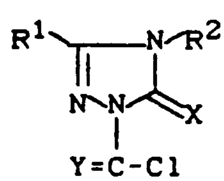

(II)

in welcher

$R^1$, $R^2$, X und Y die oben angegebene Bedeutung haben,

mit Aminen der Formel (III),

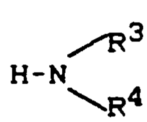

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt, oder

b) für den Fall, daß $R^3$ Wasserstoff bedeutet, wenn man in 1-Stellung unsubstituierte Triazolinone der Formel (IV),

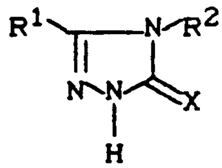

(IV)

in welcher

$R^1$, $R^2$ und X die oben angegebene Bedeutung haben,

mit Iso(thio)cyanaten der Formel (V),

$R^4$-N = C = Y (V)

in welcher

$R^4$ und Y die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich höhere herbizide Potenz gegenüber Problemunkräutern als die aus dem Stand der Technik bekannten Stickstoffheterocyclen, wie beispielsweise das Imidazolin-2-on-1-carbonsäureisobutylamid, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für einen Rest

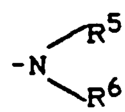

oder für einen Rest $-S(O)_n-R^7$ steht, wobei

$R^5$ und $R^6$ unabhängig voneinander jeweils für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl oder Alkoxy mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder

$R^5$ und $R^6$ gemeinsam mit dem Stickstoffatom, an welches sie gebunden sind, für einen gegebenenfalls einfach oder mehrfach gleich oder verschieden substituierten fünf- bis zehngliedrigen

Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

$R^7$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil oder Aryl mit 6 bis 10 Kohlenstoffatomen steht, wobei als Arylsubstituenten jeweils in Frage kommen: Halogen, Cyano, Nitro sowie jeweils geradkettiges oder verzweigtes Alkyl, Alkoxy oder Halogenalkyl mit jeweils 1 bis 4 Kohlenstoffatomen und gegebenenfalls 1 bis 9 gleichen oder verchiedenen Halogenatomen und

n für eine Zahl 0, 1 oder 2 steht,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenalls 1 bis 6 Kohlenstoffatomen im geradkettigen oder verzweigten Alkylteil steht, wobei als Arylsubstituenten in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ für Wasserstoff oder für geradkettiges oder verzweigtes Alkyl mit 1 bis 8 Kohlenstoffatomen steht,

$R^4$ für geradkettiges oder verzweigtes Alkoximinoalkyl mit jeweils 1 bis 8 Kohlenstoffatmen in den einzelnen Alkylteilen steht,

X für Sauerstoff oder Schwefel steht und

Y für Sauerstoff oder Schwefel steht.

Besonders bevorzugt sind Verbindungen der Formel (I), bei welchen

$R^1$ für einen Rest

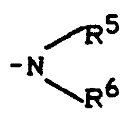

oder für einen Rest $-S(O)_n-R^7$ steht, wobei

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopentyl, Cyclohexyl, Cyclopropylmethyl, Cyhclopentylmethyl, Cyclohexylmethyl, Cyclohexylethyl, oder für jeweils gegebenenfalls ein- bis dreifach, gleich oder verschieden substituiertes Benzyl, Phenylethyl oder Phenyl stehen, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio oder

$R^5$ und $R^6$ gemeinsam mit dem Stickstoffatom, an welches sie gebunden sind, für einen gegebenenfalls ein- bis dreifach, gleich oder verschieden substituierten Heterocyclus der Formel

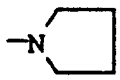 ; 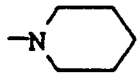 ; 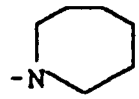 ; 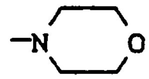 ;

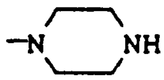 ; 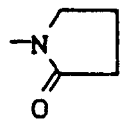 ; 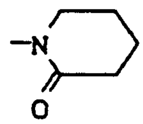 ; 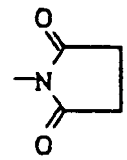 ;

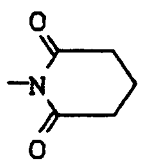 ; 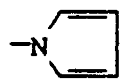 oder 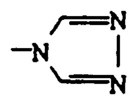 stehen,

wobei als Substituenten in Frage kommen: Methyl, Ethyl, n- oder i-Propyl, Chlor oder Trifluormethyl,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden substituiertes Benzyl oder Phenyl steht, wobei als Substituenten jeweils infrage kommen: Fluor, Chlor, Brom, Cyano, Nitro, Methyl, Ethyl, n- oder i- Propyl, n-, i-, s- oder t-Butyl, Methoxy, Ethoxy oder Trifluormethyl und

n für eine Zahl 0, 1 oder 2 steht,

$R^2$ für Methyl, Ethyl, n- oder i-Propyl, n-, i-, s-oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopropyl, Cyclopentyl, Cyclohexyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^3$ für Wasserstoff, Methyl, Ethyl, n- oder i-Propyl, n-, i-, s- oder t-Butyl steht,

$R^4$ für geradkettiges oder verzweigtes Alkoximinoalkyl mit jeweils 1 bis 5 Kohlenstoffatomen in den einzelnen Alkylteilen steht,

X für Sauerstoff oder Schwefel steht und

Y für Sauerstoff oder Schwefel steht.

Ganz besonders bevorzugt sind Verbindungen der Formel (I), bei welchen

$R^1$ für einen Rest

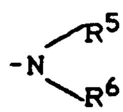

oder für einen Rest $-S(O)_n-R^7$ steht, wobei

$R^5$ und $R^6$ unabhängig voneinander jeweils für Methyl, Ethyl, n- oder i-Propyl, n-, i-, s- oder t-Butyl, für Allyl, Propargyl, Methoxymethyl, Cyclohexyl, Benzyl oder Phenyl stehen oder gemeinsam mit dem Stickstoffatom, an welches sie gebunden sind für einen Heterocyclus der Formel

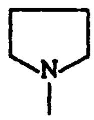 ; 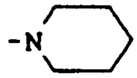 oder 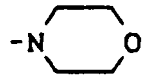 stehen,

$R^7$ für Methyl, Ethyl, n- oder i-Propyl sowie n-, i-, s- oder t-Butyl, für Allyl, Propargyl, Benzyl oder Phenyl steht und

n für O steht,

$R^2$ für Methyl, Ethyl, n- oder i-Propyl sowie n-, i-, s- oder t-Butyl, oder für Cyclohexyl, Benzyl oder Phenyl steht,

$R^3$ für Wasserstoff steht,

$R^4$ für jeweils gegebenenfalls geradkettiges oder verzweigtes Methoximinomethyl, Ethoximinomethyl, Methoximinoethyl, Ethoximinoethyl, Propoximinoethyl, Methoximinopropyl, Ethoximinopropyl, Propoximinopropyl, Methoximinobutyl, Ethoximinobutyl, Propoximinobutyl, Methoximinopentyl, Ethoximinopentyl oder Propoximinopentyl steht,

X für Sauerstoff oder Schwefel steht und

Y für Sauerstoff oder Schwefel steht.

Im einzelnen seien außer den bei den Herstellungsbeispielen genannten Verbindungen die folgenden substituierten Triazolinone der allgemeinen Formel (I) genannt:

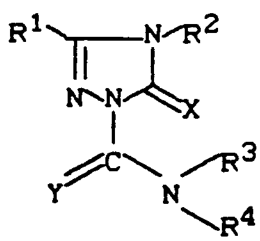 (I)

**Tabelle 1**

| $R^1$ | $R^2$ | X | Y | 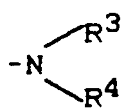 |
|---|---|---|---|---|
| 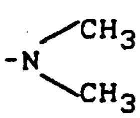 | $CH_3$ | O | O | $-NH-CH_2-CH=N-OCH_3$ |
| 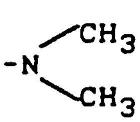 | $CH_3$ | O | O | 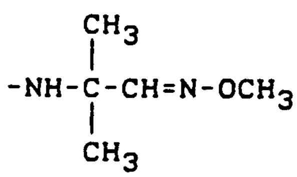 |

Tabelle 1 - Fortsetzung

| $R^1$ | $R^2$ | X | Y | 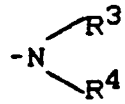 |
|---|---|---|---|---|
| 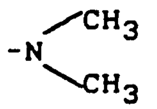 | $CH_3$ | O | O | 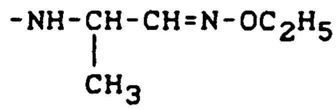 |
| 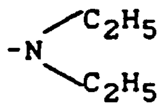 | $CH_3$ | O | O | $-NH-CH_2-CH=N-OCH_3$ |
| 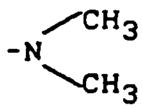 | $C_2H_5$ | O | O | 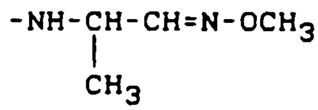 |
| 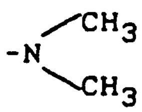 | $CH_3$ | O | O | 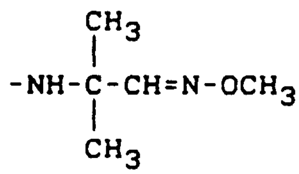 |
| 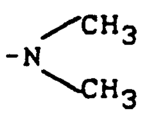 | $CH_3$ | O | O | 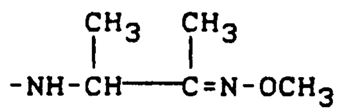 |
| 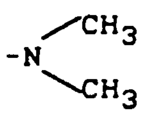 | $CH_3$ | O | O | 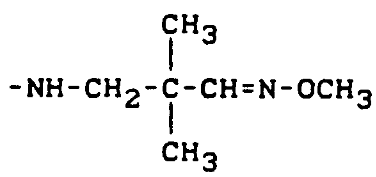 |
| 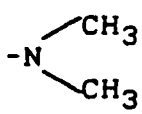 | $CH_3$ | O | O | 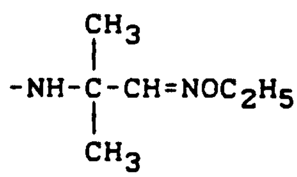 |

Tabelle 1 - Fortsetzung

| $R^1$ | $R^2$ | X | Y | 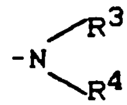 |
|---|---|---|---|---|
| $-SCH_3$ | $CH_3$ | O | O | 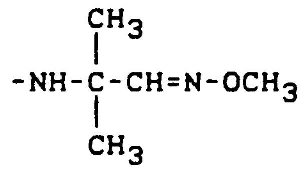 |
| $-SCH_3$ | $CH_3$ | S | O | 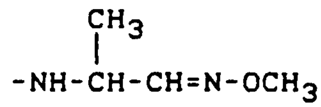 |
| 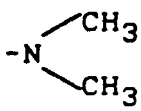 | $CH_3$ | O | O | $-NH-(CH_2)_3-CH=N-OCH_3$ |
| 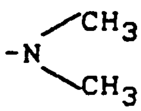 | $CH_3$ | O | O | $-NH-(CH_2)_4-CH=N-OCH_3$ |
| 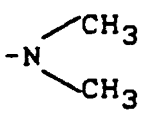 | $CH_3$ | O | O | 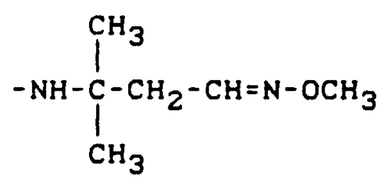 |
| 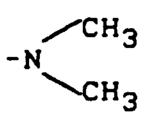 | $CH_3$ | O | O | 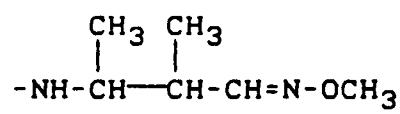 |

Verwendet man beispielsweise 1-Chlorcarbonyl-3-dimethylamino-4-methyl-1,2,4-triazolin-5-on und 1-Methyl-2-methoximino-ethylamin als Ausgangsstoffe, so läßt sich der Reaktionsablauf des erfindungsgemäßen Verfahrens (a) durch das folgende Formelschema darstellen:

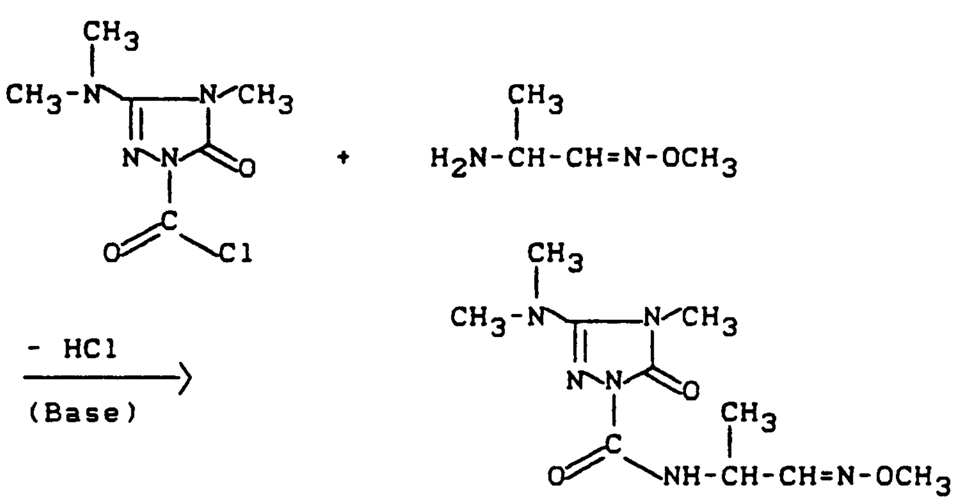

Verwendet man beispielsweise 3-Dimethylamino-4-methyl-1H-1,2,4-triazolin-5-on und 2-Methoximinoethylisocyanat als Ausgangsstoffe, so läßt sich der Reaktionsablauf des erfindungsgemäßen Verfahrens (b) durch das folgende Formelschema darstellen:

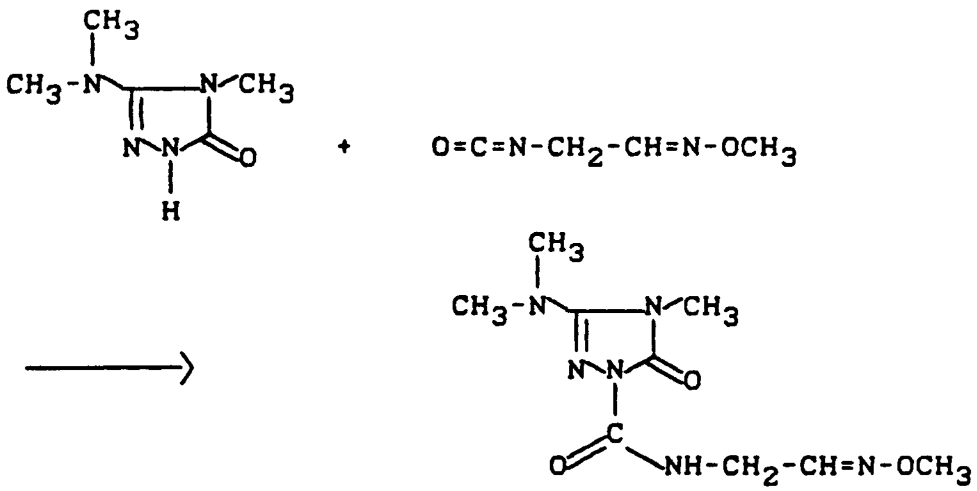

Die zur Durchführung des erfindungsgemäßen Verfahrens (a) als Ausgangsstoffe benötigten Chlor(thio)-carbonyltriazolinone sind durch die Formel (II) allgemein definiert. In dieser Formel (II) stehen $R^1$, $R^2$, X und Y vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Chlor(thio)carbonyltriazolinone der Formel (II) sind aus EP-A-0 283 876 (Stand der Technik nach Artikel 54 (3) EPÜ) bekannt.

Man erhält sie, wenn man in 1-Stellung unsubstituierte Triazolinone der Formel (IV),

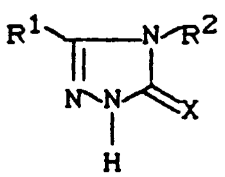

(IV)

in welcher

$R^1$, $R^2$ und X die oben angegebene Bedeutung haben,

mit (Thio)Phosgen der Formel (VI),

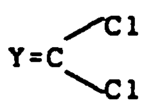

(VI)

in welcher

Y die oben angegebene Bedeutung hat,

gegebenenfalls in Gegenwart eines Verdünnungsmittels wie beispielsweise Toluol oder Acetonitril und gegebenenfalls in Gegenwart eines Säurebindemittels wie beispielsweise Triethylamin bei Temperaturen zwischen + 20 °C und + 150 °C umsetzt.

Chlor(thio)carbonylverbindungen der Formel (IIa),

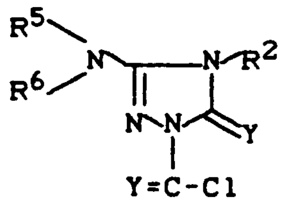

(IIa)

in welcher

$R^2$, $R^5$, $R^6$ und Y die oben angegebene Bedeutung haben,

erhält man alternativ auch, wenn man Aminoguanidiniumhydrochloride der Formel (VII),

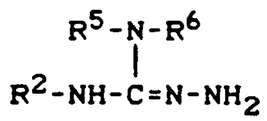

x HCl (VII)

in welcher

$R^2$, $R^5$ und $R^6$ die oben angegebene Bedeutung haben,

mit doppelt-molarem Überschuß an (Thio)Phosgen der Formel (VI),

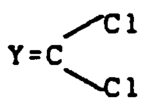

(VI)

in welcher

Y die oben angegebene Bedeutung hat,

gegebenenfalls in Gegenwart eines Verdünnungsmittels wie beispielsweise Toluol oder Acetonitril und gegebenenfalls in Gegenwart eines Säurebindemittels wie beispielsweise Triethylamin bei Temperaturen zwischen +20 °C und + 150 °C umsetzt.

Die Aminoguanidiniumhydrochloride der Formel (VII) erhält man in Analogie zu bekannten Verfahren, beispielsweise wenn man die allgemein bekannten Harnstoffe der Formel (VIII),

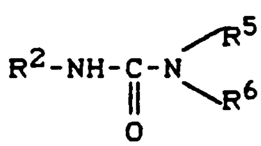

(VIII)

in welcher

$R^2$, $R^5$ und $R^6$ die oben angegebene Bedeutung haben,

zunächst in einer 1. Stufe mit (Thio)Phosgen der Formel (VI),

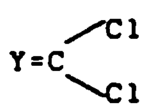

(VI)

in welcher

Y die oben angegebene Bedeutung hat,

gegebenenfalls in Gegenwart eines Verdünnungsmittels wie beispielsweise Toluol oder Acetonitril bei Temperaturen zwischen + 10 °C und + 150 °C umsetzt, und die so erhältlichen Formamidinhydrochloride

der Formel (IX),

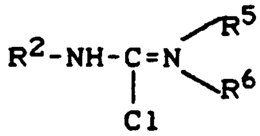 x HCl (IX)

in welcher

$R^2$, $R^5$ und $R^6$ die oben angegebene Bedeutung haben,

in einer 2. Stufe mit Hydrazinhydrat, gegebenenfalls in Gegenwart eines Verdünnungsmittels wie beispielsweise Isopropanol oder Dichlormethan bei Temperaturen zwischen - 10 °C und + 60 °C umsetzt (vgl. z.B. J. org. Chem. 19, 1807 [1954]; Bull. Soc. Chim. Fr. 1975, 1649; US 2 845 458).

Hernstoffe der Formel (VIII), Phosgen und Thiophosgen der Formel (VI) sind allgemein bekannte Verbindungen der organischen Chemie.

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind bekannt oder erhältlich in Analogie zu bekannten Verfahren (vgl. z. B. DE-OS 33 06 197).

Die zur Durchführung des erfindungsgemäßen Verfahrens (b) und zur Synthese der Vorprodukte der Formel (II) als Ausgangsstoffe benötigten in 1-Stellung unsubstituierten Triazolinone sind durch die Formel (IV) allgemein definiert. In dieser Formel (IV) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolinone der Formel (IV) sind teilweise bekannt (vgl. z. B. Chem. Ber. 102, 735 [1969]; Chem. Ber. 107, 454 [1974]; Arch. Phar. 307, 509 [1974]; Helv. Chim. Acta 63, 841 [1980]; US 4 098 896; US 4 110 332; US 4 530 898; DE-OS 22 50 572; J. Chem. Soc. C. 1967, 746; J. Chem. Soc. Perkin Trans. I, 1059 [1982]; Arzneimittel Forsch. 27, 343 [1977]; Compt. Rend. 253, 1974 [1961]; Bull. Soc. Chim. Fr. 1963, 144; French Patent FR M 1559 vom 3.12.62). Man erhält die bekannten, ebenso wie die nicht bekannten Verbindungen der Formel (IV) in Analogie zu bekannten Verfahren (vgl. z. B. J. org. Chem. 51, 1719 [1986]; US 4 098 896 sowie die Herstellungsbeispiele).

In 1-Stellung unsubstituierte Triazolinone der Formel (IVa),

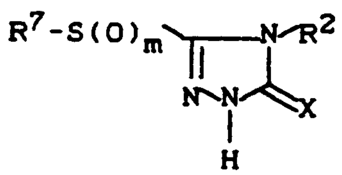 (IVa)

in welcher

$R^2$, $R^7$ und X die oben angegebene Bedeutung haben und

m für eine Zahl 1 oder 2 steht,

erhält man aus den entsprechenden Verbindungen der Formel (IVb),

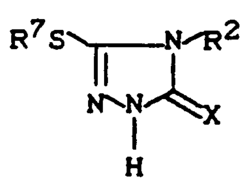 (IVb)

in welcher

$R^2$, $R^7$ und X die oben angegebene Bedeutung haben,
in allgemein bekannter Art und Weise mit üblichen Oxidationsmitteln, beispielsweise durch Umsetzung mit 3-Chlorperbenzoesäure gegebenenfalls in Gegenwart eines Verdünnungsmittels wie beispielsweise Dichlormethan oder Acetonitril und gegebenenfalls in Gegenwart eines Katalysators wie beispielsweise Ammoniummolybdat bei Temperaturen zwischen 0 °C und 40 °C.

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen $R^4$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (V) sind nocht nicht bekannt.
Man erhält sie in Analogie zu bekannten Verfahren (vgl. z. B. Saul Patai "The Chemistry of Cyanates and their Thioderivatives", John Wiley & Sons, New York 1977), beispielsweise wenn man Amine der Formel (III),

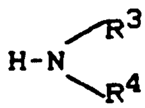 (III)

in welcher
$R^3$ und $R^4$ die oben angegebenen Bedeutungen haben,
mit (Thio)phosgen der Formel (VI),

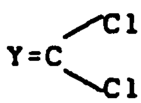 (VI)

in welcher
Y die oben angegebene Bedeutung hat,
gegebenenfalls in Gegenwart eines Verdünnungsmittels wie beispielsweise Toluol oder Chloroform und gegebenenfalls in Gegenwart eines Säurebindemittel wie beispielsweise Pyridin oder Triethylamin bei Temperaturen zwischen 20 °C und 250 °C umsetzt.

Als Verdünnungsmittel zur Durchführung des erfindungsgemäßen Verfahrens (a) kommen vorzugsweise inerte organische Lösungsmittel infrage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen wie Pyridin.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundercen (DBU).

Es ist auch möglich das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temepraturen zwischen 0 °C und + 150 °C, vorzugsweise bei Temperaturen zwischen + 10 °C und + 80 °C.

Das erfindungsgemäße Verfahren (a) wird üblicherweise unter Normaldruck durchgerführt. Es ist jedoch auch möglich unter erhöhtem Druck zu arbeiten.

Zur Durchführung des erfindungsgemäßen Verfahrens (a) setzt man pro Mol an 1-Chlor-(thio)carbonyl-triazolinon der Formel (II) im allgemeinen 1,0 bis 5,0 Mol, vorzugsweise 1,0 bis 2,5 Mol am Amin der Formel (III) und gegebenenfalls 1,0 bis 2,5 Mol an Säurebindemittel ein. Die Reaktionsdurchführung, Aufarbeitung und Isolierung der Reaktionsprodukte erfolgt in Analogie zu allgemein bekannten Verfahren.

Als Verdünnungsmittel zur Durchführung des erfindungsgemäßen Verahrens (b) kommen ebenfalls inerte organische Lösungsmittel infrage. Vorzugsweise verwendet man die bei Verfahren (a) genannten Verdünnungsmittel.

Das erfindungsgemäße Verfahren (b) kann gegebenenfalls in Gegenwart eines basischen Reaktionshilfsmittels durchgeführt werden. Als solche kommen alle üblichen anorganischen und organischen Basen infrage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Der Zusatz solcher Katalysatoren ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und + 150 °C, vorzugsweise bei Temperaturen zwischen + 40 °C und + 120 °C.

Das erfindungsgemäße Verfahren (b) wird üblicherweise unter Normaldruck durchgeführt. Es ist jedoch auch möglich unter erhöhtem Druck zu arbeiten.

Zur Durchführung des erfindungsgemäßen Verfahrens (b) setzt man pro Mol an in 1-Stellung unsubstituiertem Triazolinon der Formel (IV) im allgemeinen 1,0 bis 5,0 Mol, vorzugsweise 1,0 bis 2,5 Mol an Iso-(thio)cyanat der Formel (V) und gegebenenfalls 1,0 bis 2,5 Mol an Reaktionshilfsmittel ein. Die Reaktionsdurchführung, Aufarbeitung und Isolierung der Reaktionsprodukte erfolgt in Analogie zu allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>
Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keinswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung dikotyler Unkräuter insbesondere in monokotylen Kulturen wie beispielsweise Weizen oder Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch oprganische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohl-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit 2,4-Dichlorphenoxyessigsäure; 4-(2,4-Dichlorphenoxy)-buttersäure; 2,4-Dichlorphenoxypropionsäure; 2-Chlor-4-ethylamino-6-isopropylamino-1,3,5-triazin; 3-Isopropyl-2,1,3-benzothiadiazin-4-on-2,2-dioxid; Methyl-5-(2,4-dichlorphenoxy)-2-nitrobenzoat; 3,5-Dibrom-4-hydroxy-benzonitril; 2-Chlor-N-{[(4-methoxy-6-methyl-1,3,5-triazin-2-yl)-amino]-carbonyl}-benzolsulfonamid; N,N-Dimethyl-N′-(3-chlor-4-methylphenyl)-harnstoff; 2-Chlor-4-ethyl-amino-6-(3-cyanopropylamino)-1,3,5-triazin; 2-[4-(2,4-Dichlorphenoxy)-phenoxy]-propionsäure, deren Methyl-oder deren Ethylester; 3,6-Dichlor-2-pyridincarbonsäure; N,N-Di-n-propyl-thiocarbamidsäure-S-ethylester; 4-Amino-6-t-butyl-3-ethylthio-1,2,4-triazin-5(4H)-on; 2-{4-[(6-Chlor-2-benzoxazolyl)-oxy]-phenoxy}-propansäure, deren Methyl- oder deren Ethylester; 2-[4-(3,5-Dichlorpyrid-2-yloxy)-phenoxy]-propionsäure-(trimethylsilylmethylester); Methyl-2-[4,5-dihydro-4-methyl-4-(1-methylethyl)-5-oxo-1H-imidazol-2-yl]-4(5)-methylbenzoat; N,N-Dimethyl-N′-(4-isopropylphenyl)-harnstoff; (2-Methyl-4-chlorphenoxy)-essigsäure; (4-Chlor-2-methylphenoxy)-propionsäure; N-Methyl-2-(1,3-benzthiazol-2-yloxy)-acetanilid; 2-{[[((4-Methoxy-6-methyl-1,3,5-triazin-2-yl)-amino)-carbonyl]-amino]-sulfonyl}-benzoesäure oder deren Methylester; N-(1-Ethylpropyl)-3,4-dimethyl-2,6-dinitroanilin; O-(6-Chlor-3-phenyl-pyridazin-4-yl)-S-octylthiocarbonat; 4-Ethylamino-2-t-butylamino-6-methylthio-s-triazin; 3-[[[[(4-Methoxy-6-methyl-1,3,5-triazin-2-yl)-amino]-carbonyl]-amino]-sulfonyl]-thiophen-2-carbonsäure-methylester; N,N-Diisopropyl-S-(2,3,3-trichlorallyl)-thiocarbamat und 3,5,6-Trichlor-2-pyridyloxyessigsäure sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln

ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Herstellungsbeispiele

Beispiel 1

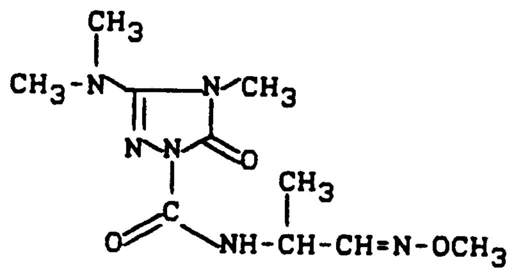

(Verfahren (a))

Zu 4 g (0.02 Mol) 1-Chlorcarbonyl-3-dimethylamino-4-methyl-1,2,4-triazolin-5-on in 100 ml Dichlormethan tropft man unter Rühren 4,1 g (0.04 Mol) 1-Methoximino-2-propylamin so zu, daß die Reaktionstemperatur 40 °C nicht übersteigt. Nach beendeter Zugabe rührt man 12 Stunden bei Raumtemperatur, wäscht dann zweimal mit jeweils 100 ml Wasser, trocknet über Natriumsulfat und entfernt das Lösungsmittel im Vakuum.

Man erhält 3,4 g (63 % der Theorie) an 1-[N-(1-Methoximino-2-propyl)-aminocarbonyl]-3-dimethylamino-4-methyl-1,2,4-triazolin-5-on als Öl.
$^1$H-NMR ($CDCl_3$/TMS) : $\delta$ = 3.85 ppm ($OCH_3$).

Herstellung der Ausgangsverbindungen

Beispiel II-1

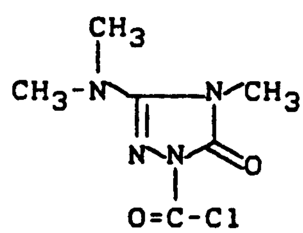

71 g (0,5 Mol) 3-Dimethylamino-4-methyl-1H-1,2,4-triazolin-5-on in 300 ml Toluol werden unter Einleiten von Phosgen auf 120 °C erwärmt. Insgesamt leitet man 115 g (1,15 Mol) Phosgen ein. Ab 80 °C findet eine lebhafte Chlorwasserstoffentwicklung statt. Nach beendeter Phosgen-Einleitung rührt man weitere 5 Stunden bei 120 °C, entfernt überschüssiges Phosgen und Chlorwasserstoff durch Ausblasen mit Stickstoff und filtriert die Mischung bei 20 °C. Das Filtrat wird mit 1 l Cyclohexan verrührt, das ausgefallene Produkt

abgesaugt, mit Cyclohexan gewaschen und getrocknet.

Man erhält 70 g (69 % der Theorie) an 1-Chlorcarbonyl-3-dimethylamino-4-methyl-1,2,4-triazolin-5-on vom Schmelzpunkt 78 °C - 80 °C.

Beispiel IV-1

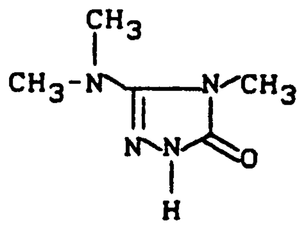

In eine Suspension aus 152,5 g (1 Mol) 1-Amino-2,2,3-trimethylguanidiniumhydrochlorid in 1000 ml Acetonitril leitet man innerhalb von 2 Stunden unter Rühren bei 80 °C 150 g (1,5 Mol) Phosgen, rührt 30 MInuten bei 80 °C nach, kühlt auf 20 °C ab, entfernt überschüssiges Phosgen durch Ausblasen mit Stickstoff, saugt ausgefallenes Produkt ab, löst es in 1000 ml Wasser, neutralisiert mit konzentrierter Natronlauge und engt im Vakuum zur Trockne ein. Der ölige Rückstand wird in 1000 ml Acetonitril aufgenommen, filtriert und das Filtrat im Vakuum vom Lösungsmittel befreit.

Man erhält 80 g (57 % der Theorie) an 3-Dimethylamino-4-methyl-1H-1,2,4-triazolin-5-on vom Schmelzpunkt 78 °C - 80 °C.

Beispiel VII-1

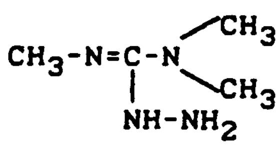 x HCl

Zu 50 g (1 Mol) Hydrazinhydrat in 300 ml Isopropanol tropft man bei 20 °C bis 25 °C unter Rühren innerhalb von 30 Minuten eine Lösung von 78,5 g (0,5 Mol) Chlortrimethylformamidiniumhydrochlorid in 250 ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

Beispel IX-1

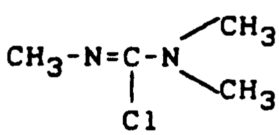 x HCl

In eine Mischung aus 510 g (5 Mol) N,N,N′-trimethylharnstoff und 3 l Chlorbenzol leitet man bei 80 °C innerhalb von 2,5 Stunden unter Rühren 545 g (5,5 Mol) Phosgen und rührt nach beendetem Einleiten weitere 45 Minuten bis zum Ende der Kohlendioxid-Entwicklung bei 80 °C nach. Die Reaktionsmischung wird abgekühlt auf 10 °C, das wasserempfindliche Produkt unter Stickstoff abgesaugt, mit 1 l Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76 °C bis 78 °C.

In entsprechender Weise und gemäß den allgemeinen Angaben zur Herstellung erhält man die folgenden substituierten Triazolinone der allgemeinen Formel (I):

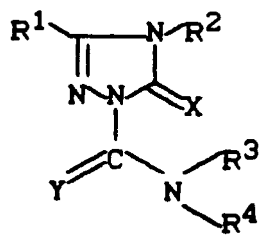

(I)

Tabelle 2

| Bsp.-Nr. | $R^1$ | $R^2$ | X | Y | 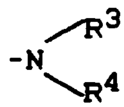 | physikalische Eigenschaften |
|---|---|---|---|---|---|---|
| 2 | $CH_3S-$ | $CH_3$ | O | O | 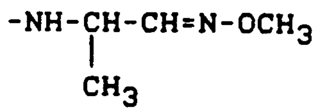 | $^1$H-NMR*): 3.8 |
| 3 | $(CH_3)_2N-$ | $CH_3$ | O | O | $-NH-CH_2-CH=N-OCH_3$ | Öl |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiel

In dem folgenden Anwendungsbeispiel wurde die nachstehend aufgeführte Verbindung als Vergleichsubstanz eingesetzt:

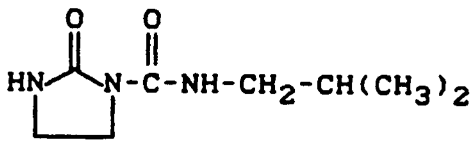 (A)

Imidazolidin-2-on-1-carbonsäureisobutylamid

(bekannt aus K. H. Büchel, "Pflanzenschutz und Schädlingsbekämpfung" S. 170, Thieme Verlag Stuttgart 1977)

<u>Beispiel A</u>

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigt in diesem Test beispielsweise die Verbindung gemäß Herstellungsbeispiel 1.

**Patentansprüche**

**1.** Substituierte Triazolinone der allgemeinen Formel (I),

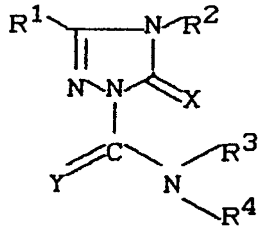 (I)

in welcher
$R^1$ für einen Rest

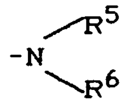

oder für einen Rest $-S(O)_n-R^7$ steht, wobei

$R^5$ und $R^6$ unabhängig voneinander jeweils für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl oder Alkoxy mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder

$R^5$ und $R^6$ gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

$R^7$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil oder Aryl mit 6 bis 10 Kohlenstoffatomen steht, wobei als Arylsubstituenten jeweils in Frage kommen: Halogen, Cyano, Nitro sowie jeweils geradkettiges oder verzweigtes Alkyl, Alkoxy oder Halogenalkyl mit jeweils 1 bis 4 Kohlenstoffatomen und gegebenenfalls 1 bis 9 gleichen oder verchiedenen Halogenatomen und

n für eine Zahl 0, 1 oder 2 steht,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ für Wasserstoff oder für geradkettiges oder verzweigtes Alkyl mit 1 bis 8 Kohlenstoffatomen steht,
$R^4$ für geradkettiges oder verzweigtes Alkoximinoalkyl mit jeweils 1 bis 8 Kohlenstoffatomen in den einzelnen Alkylteilen steht,
X für Sauerstoff oder Schwefel steht und
Y für Sauerstoff oder Schwefel steht.

**2.** Verfahren zur Herstellung von substituierten Triazolinonen der allgemeinen Formel (I),

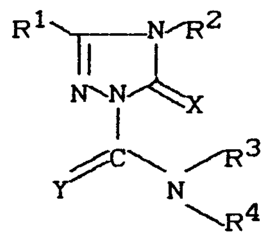

(I)

in welcher
$R^1$ für einen Rest

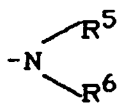

oder für einen Rest $-S(O)_n-R^7$ steht, wobei
$R^5$ und $R^6$ unabhängig voneinander jeweils für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl oder Alkoxy mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder
$R^5$ und $R^6$ gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,
$R^7$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils

gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil oder Aryl mit 6 bis 10 Kohlenstoffatomen steht, wobei als Arylsubstituenten jeweils in Frage kommen: Halogen, Cyano, Nitro sowie jeweils geradkettiges oder verzweigtes Alkyl, Alkoxy oder Halogenalkyl mit jeweils 1 bis 4 Kohlenstoffatomen und gegebenenfalls 1 bis 9 gleichen oder verchiedenen Halogenatomen und

n für eine Zahl 0, 1 oder 2 steht,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ für Wasserstoff oder für geradkettiges oder verzweigtes Alkyl mit 1 bis 8 Kohlenstoffatomen steht,

$R^4$ für geradkettiges oder verzweigtes Alkoximinoalkyl mit jeweils 1 bis 8 Kohlenstoffatomen in den einzelnen Alkylteilen steht,

X für Sauerstoff oder Schwefel steht und

Y für Sauerstoff oder Schwefel steht,

dadurch gekennzeichnet, daß man

a) 1-Chlor-(thio)-carbonyltriazolinone der Formel (II),

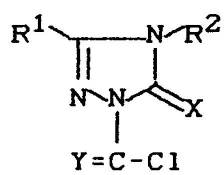

(II)

in welcher

$R^1$, $R^2$, X und Y die oben angegebene Bedeutung haben,

mit Aminen der Formel (III),

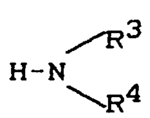

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt, oder

b) für den Fall, daß $R^3$ Wasserstoff bedeutet, wenn man in 1-Stellung unsubstituierte Triazolinone der Formel (IV),

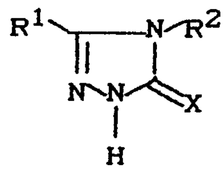

(IV)

in welcher

$R^1$, $R^2$ und X die oben angegebene Bedeutung haben,

mit Iso(thio)cyanaten der Formel (V),

$R^4$-N = C = Y (V)

in welcher

$R^4$ und Y die oben angegebene Bedeutung haben,

gegebenenfalls in Gegenwart eines Verdünnungsmittels und gegebenenfalls in Gegenwart eines Reaktionshilfsmittels umsetzt.

**3.** Herbizide Mittel, gekennzeichnet durch einen Gehalt an mindestens einem substituierten Triazolinon der Formel (I) gemäß den Ansprüchen 1 und 2.

**4.** Verfahren zur Bekämpfung von Unkräutern, dadurch gekennzeichnet, daß man substituierte Triazolinone der Formel (I) gemäß den Ansprüchen 1 und 2 auf die Unkräuter und/oder ihren Lebensraum einwirken läßt.

**5.** Verwendung von substituierten Triazolinonen der Formel (I) gemäß den Ansprüchen 1 und 2 zur Bekämpfung von Unkräutern.

**6.** Verfahren zur Herstellung von herbiziden Mitteln, dadurch gekennzeichnet, daß man substituierte Triazolinone der Formel (I) gemäß den Ansprüchen 1 und 2 mit Streckmitteln und/oder oberflächenaktiven Substanzen vermischt.

## Claims

**1.** Substituted triazolinones of the general formula (I)

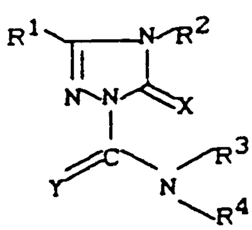

(I)

in which

$R^1$ represents an

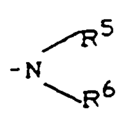

radical or an $-S(O)_n-R^7$ radical, in which

$R^5$ and $R^6$ independently of one another in each case represent straight-chain or branched alkyl having 1 to 8 carbon atoms, alkenyl having 2 to 8 carbon atoms, alkinyl having 2 to 8

carbon atoms, halogenoalkyl having 1 to 8 carbon atoms and 1 to 17 identical or different halogen atoms, halogenoalkenyl or halogenoalkinyl each having 2 to 8 carbon atoms and 1 to 15 or 13 identical or different halogen atoms, alkoxyalkyl or alkoxy each having 1 to 6 carbon atoms in the individual alkyl moieties, cycloalkyl having 3 to 7 carbon atoms, cycloalkylalkyl having 3 to 7 carbon atoms in the cycloalkyl moiety and 1 to 6 carbon atoms in the alkyl moiety or aralkyl having 6 to 10 carbon atoms in the aryl moiety and 1 to 6 carbon atoms in the alkyl moiety, aryl having 6 to 10 carbon atoms or heteroaryl having 2 to 9 carbon atoms and 1 to 3 hetero atoms, in particular nitrogen, oxygen and/or sulphur, which are in each case optionally mono-substituted or polysubstituted by identical or different substituents, suitable substituents in each case being: halogen, cyano, nitro and also in each case straight-chain or branched alkyl, alkoxy, alkylthio, halogenoalkyl, halogenoalkoxy or halogenoalkylthio each having 1 to 4 carbon atoms and, where appropriate, 1 to 9 identical or different halogen atoms, or

$R^5$ and $R^6$, together with the nitrogen atom to which they are bonded, represent a five- to ten-membered heterocyclic ring, which can optionally contain 1 to 2 additional hetero atoms, in particular nitrogen, oxygen and/or sulphur, and is optionally monosubstituted or polysubstituted by identical or different substituents, suitable substituents being: halogen and also in each case straight-chain or branched alkyl or halogenoalkyl each having 1 to 4 carbon atoms and, where appropriate, 1 to 9 identical or different halogen atoms and also 1 to 2 oxo or thiono groups,

$R^7$ represents in each case straight-chain or branched alkyl having 1 to 8 carbon atoms, alkenyl having 2 to 8 carbon atoms, alkinyl having 2 to 8 carbon atoms, cycloalkyl having 3 to 7 carbon atoms, cycloalkylalkyl having 3 to 7 carbon atoms in the cycloalkyl moiety and 1 to 6 carbon atoms in the alkyl moiety or aralkyl having 6 to 10 carbon atoms in the aryl moiety and 1 to 6 carbon atoms in the alkyl moiety or aryl having 6 to 10 carbon atoms, which are each optionally monosubstituted or polysubstituted by identical or different substituents, suitable aryl substituents in each case being: halogen, cyano, nitro and also in each case straight-chain or branched alkyl, alkoxy or halogenoalkyl each having 1 to 4 carbon atoms and, where appropriate, 1 to 9 identical or different halogen atoms, and

n represents the number 0, 1 or 2,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy each having 1 to 6 carbon atoms in the individual alkyl moieties, cycloalkylalkyl or cycloalkyl each having 3 to 7 carbon atoms in the cycloalkyl moiety and, where appropriate, 1 to 6 carbon atoms in the straight-chain or branched alkyl moiety or aralkyl or aryl each having 6 to 10 carbon atoms in the aryl moiety and, where appropriate, 1 to 6 carbon atoms in the straight-chain or branched alkyl moiety, each of which is optionally monosubstituted or polysubstituted by identical or different substituents, suitable aryl substituents in each case being: halogen, cyano, nitro, and also in each case straight-chain or branched alkyl, alkoxy, alkylthio, halogenoalkyl, halogenoalkoxy or halogenoalkylthio each having 1 to 4 carbon atoms and, where appropriate, 1 to 9 identical or different halogen atoms,

$R^3$ represents hydrogen or straight-chain or branched alkyl having 1 to 8 carbon atoms,

$R^4$ represents straight-chain or branched alkoximinoalkyl each having 1 to 8 carbon atoms in the individual alkyl moieties,

X represents oxygen or sulphur and

Y represents oxygen or sulphur.

**2.** Process for the preparation of substituted triazolinones of the general formula (I)

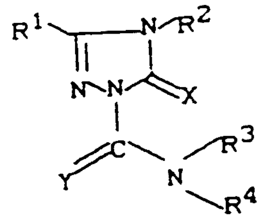

(I)

in which

R1 represents an

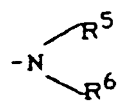

radical or an $-S(O)_n-R^7$ radical, in which

$R^5$ and $R^6$ independently of one another in each case represent straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl each having 2 to 8 carbon atoms and 1 to 15 or 13 identical or different halogen atoms, alkoxyalkyl or alkoxy each having 1 to 6 carbon atoms in the individual alkyl moieties, cycloalkyl having 3 to 7 carbon atoms, cycloalkylalkyl having 3 to 7 carbon atoms in the cycloalkyl moiety and 1 to 6 carbon atoms in the alkyl moiety or aralkyl having 6 to 10 carbon atoms in the aryl moiety and 1 to 6 carbon atoms in the alkyl moiety, aryl having 6 to 10 carbon atoms or heteroaryl having 2 to 9 carbon atoms and 1 to 3 hetero atoms, in particular nitrogen, oxygen and/or sulphur, which are in each case optionally mono-substituted or polysubstituted by identical or different substituents, suitable substituents in each case being: halogen, cyano, nitro and also in each case straight-chain or branched alkyl, alkoxy, alkylthio, halogenoalkyl, halogenoalkoxy or halogenoalkylthio each having 1 to 4 carbon atoms and, where appropriate, 1 to 9 identical or different halogen atoms, or

$R^5$ and $R^6$, together with the nitrogen atom to which they are bonded, represent a five- to ten-membered heterocyclic ring, which can optionally contain 1 to 2 additional hetero atoms, in particular nitrogen, oxygen and/or sulphur, and is optionally monosubstituted or polysubstituted by identical or different substituents, suitable substituents being: halogen and also in each case straight-chain or branched alkyl or halogenoalkyl each having 1 to 4 carbon atoms and, where appropriate, 1 to 9 identical or different halogen atoms and also 1 to 2 oxo or thiono groups,

$R^7$ represents in each case straight-chain or branched alkyl having 1 to 8 carbon atoms, alkenyl having 2 to 8 carbon atoms, alkinyl having 2 to 8 carbon atoms, cycloalkyl having 3 to 7 carbon atoms, cycloalkylalkyl having 3 to 7 carbon atoms in the cycloalkyl moiety and 1 to 6 carbon atoms in the alkyl moiety or aralkyl having 6 to 10 carbon atoms in the aryl moiety and 1 to 6 carbon atoms in the alkyl moiety or aryl having 6 to 10 carbon atoms, which are each optionally monosubstituted or polysubstituted by identical or different substituents, suitable aryl substituents in each case being: halogen, cyano, nitro and also in each case straight-chain or branched alkyl, alkoxy or halogenoalkyl each having 1 to 4 carbon atoms and, where appropriate, 1 to 9 identical or different halogen atoms, and

n represents the number 0, 1 or 2,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to IS identical or different halogen

atoms, halogenoalkinyl having 2 to 8 carbon atoms and 1 to 13 identical or different halogen atoms, alkoxyalkyl or alkoxy each having 1 to 6 carbon atoms in the individual alkyl moieties, cycloalkylalkyl or cycloalkyl each having 3 to 7 carbon atoms in the cycloalkyl moiety and, where appropriate, 1 to 6 carbon atoms in the straight-chain or branched alkyl moiety or aralkyl or aryl each having 6 to 10 carbon atoms in the aryl moiety and, where appropriate, 1 to 6 carbon atoms in the straight-chain or branched alkyl moiety, each of which is optionally monosubstituted or polysubstituted by identical or different substituents, suitable aryl substituents in each case being: halogen, cyano, nitro, and also in each case straight-chain or branched alkyl, alkoxy, alkylthio, halogenoalkyl, halogenoalkoxy or halogenoalkylthio each having 1 to 4 carbon atoms and, where appropriate, 1 to 9 identical or different halogen atoms,

$R^3$ represents hydrogen or straight-chain or branched alkyl having 1 to 8 carbon atoms,

$R^4$ represents straight-chain or branched alkoximinoalkyl each having 1 to 8 carbon atoms in the individual alkyl moieties,

X represents oxygen or sulphur and

Y represents oxygen or sulphur,

characterised in that

a) 1-chloro-(thio)-carbonyltriazolinones of the formula (II)

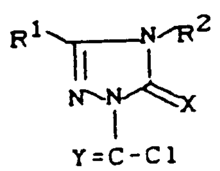

(II)

in which

$R^1$, $R^2$, X and Y have the abovementioned meaning,

are reacted with amines of the formula (III)

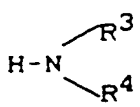

(III)

in which

$R^3$ and $R^4$ have the abovementioned meaning,

if appropriate in the presence of a diluent and if appropriate in the presence of an acid-binding agent, or

b) for the case in which $R^3$ denotes hydrogen, 1-unsubstituted triazolinones of the formula (IV)

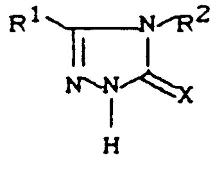

(IV)

in which

$R^1$, $R^2$ and X have the abovementioned meaning, are reacted with iso(thio)cyanates of the formula (V)

$R^4$-N=C=Y (V)

in which

$R^4$ and Y have the abovementioned meaning,

if appropriate in the presence of a diluent and if appropriate in the presence of a reaction auxiliary.

3. Herbicidal agents, characterised in that they contain at least one substituted triazolinone of the formula (I) according to Claims 1 and 2.

4. Method for combating weeds, characterised in that substituted triazolinones of the formula (I) according to Claims 1 and 2 are allowed to act on the weeds and/or their environment.

5. Use of substituted triazolinones of the formula (I) according to Claims 1 and 2 for combating weeds.

6. Process for the production of herbicidal agents, characterised in that substituted triazolinones of the formula (I) according to Claims 1 and 2 are mixed with extenders and/or surface-active substances.

**Revendications**

1. Triazolinones substituées de formule générale (I)

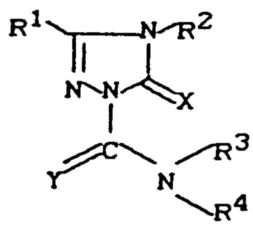

(I)

dans laquelle

$R^1$ représente un reste

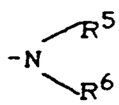

ou un reste $-S(O)_n-R^7$, où

$R^5$ et $R^6$ représentent chacun, indépendamment l'un de l'autre, un groupe, à chaîne droite ou ramifiée, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ou halogénalcynyle ayant chacun 2 à 8 atomes de carbone et 1 à 15 ou respectivement 13 atomes d'halogènes identiques ou différents, alkoxyalkyle ou alkoxy ayant chacun 1 à 6 atomes de carbone dans les parties alkyle individuelles, un groupe cyclo-alkyle ayant 3 à 7 atomes de carbone, un groupe cycloalkylalkyle ayant 3 à 7 atomes de carbone dans la partie cycloalkyle et 1 à 6 atomes de carbone dans la partie alkyle ou un groupe, portant éventuellement dans chaque cas un ou plusieurs substituants identiques ou différents, aralkyle ayant 6 à 10 atomes de carbone dans la partie aryle et 1 à 6 atomes de carbone dans la partie alkyle, aryle ayant 6 à 10 atomes de carbone ou hétéroaryle ayant 2 à 9 atomes de carbone et 1 à 3 hétéroatomes, notamment d'azote, d'oxygène et/ou de soufre, et on considère dans chaque cas comme substituants : un halogène, un radical cyano, nitro ainsi qu'un radical, à chaîne droite ou ramifiée dans chaque cas, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents, ou bien

$R^5$ et $R^6$ forment conjointement avec l'atome d'azote auquel ils sont liés un hétérocycle pentagonal à décagonal portant éventuellement un ou plusieurs substituants identiques ou différents, qui peut comporter le cas échéant 1 ou 2 autres hétéroatomes, notamment

d'azote, d'oxygène et/ou de soufre, et on considère alors comme substituants : un halogène ainsi qu'un groupe, à chaîne droite ou ramifiée dans chaque cas, alkyle ou halogénalkyle ayant chacun 1 à 4 atomes de carbone et le cas échéant 1 à 9 atomes d'halogènes identiques ou différents ainsi que 1 ou 2 groupes oxo ou thiono,

$R^7$ désigne un groupe, à chaîne droite ou ramifiée dans chaque cas, alkyle ayant 1 à 8 atomes de carbone, alcényle ayant 2 à 8 atomes de carbone, alcynyle ayant 2 à 8 atomes de carbone, un groupe cycloalkyle ayant 3 à 7 atomes de carbone, un groupe cycloalkylalkyle ayant 3 à 7 atomes de carbone dans la partie cycloalkyle et 1 à 6 atomes de carbone dans la partie alkyle ou un groupe, portant éventuellement dans chaque cas un ou plusieurs substituants identiques ou différents, aralkyle ayant 6 à 10 atomes de carbone dans la partie aryle et 1 à 6 atomes de carbone dans la partie alkyle, ou aryle ayant 6 à 10 atomes de carbone, et on considère alors dans chaque cas comme substituants du groupe aryle : un halogène, un radical cyano, nitro, ainsi qu'un radical, à chaîne droite ou ramifiée dans chaque cas, alkyle, alkoxy ou halogénalkyle ayant chacun 1 à 4 atomes de carbone et le cas échéant 1 à 9 atomes d'halogènes identiques ou différents et

n a la valeur 0, 1 ou 2,

$R^2$ désigne un groupe, à chaîne droite ou ramifiée dans chaque cas, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, un groupe cycloalkylalkyle ou cycloalkyle ayant chacun 3 à 7 atomes de carbone dans la partie cycloalkyle et le cas échéant 1 à 6 atomes de carbone dans la partie alkyle à chaîne droite ou ramifiée ou un groupe, portant éventuellement dans chaque cas un ou plusieurs substituants identiques ou différents, aralkyle ou aryle ayant chacun 6 à 10 atomes de carbone dans la partie aryle et le cas échéant 1 à 6 atomes de carbone dans la partie alkyle à chaîne droite ou ramifiée, et on considère alors dans chaque cas comme substituants de la partie aryle : un halogène, un radical cyano, nitro ainsi qu'un radical, à chaîne droite ou ramifiée dans chaque cas, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents,

$R^3$ est l'hydrogène ou un groupe alkyle à chaîne droite ou ramifiée ayant 1 à 8 atomes de carbone,

$R^4$ est un groupe alkoximinoalkyle à chaîne droite ou ramifiée ayant 1 à 8 atomes de carbone dans les parties alkyle individuelles,

X est l'oxygène ou le soufre et

Y est l'oxygène ou le soufre.

**2.** Procédé de production de triazolinones substituées de formule générale (I)

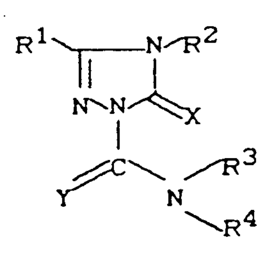

(I)

dans laquelle

$R^1$ représente un reste

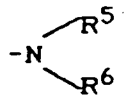

ou un reste $-S(O)_nR^7$, où

$R^5$ et $R^6$ représentent chacun, indépendamment l'un de l'autre, un groupe, dans chaque cas à chaîne droite ou ramifiée, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ou halogénalcynyle ayant chacun 2 à 8 atomes de carbone et 1 à 15 ou respectivement 13 atomes d'halogènes identiques ou différents, alkoxyalkyle ou alkoxy ayant chacun 1 à 6 atomes de carbone dans les parties alkyle individuelles, un groupe cycloalkyle ayant 3 à 7 atomes de carbone, un groupe cycloalkylalkyle ayant 3 à 7 atomes de carbone dans la partie cycloalkyle et 1 à 6 atomes de carbone dans la partie alkyle ou un groupe, portant éventuellement dans chaque cas un ou plusieurs substituants identiques ou différents, aralkyle ayant 6 à 10 atomes de carbone dans la partie aryle et 1 à 6 atomes de carbone dans la partie alkyle, aryle ayant 6 à 10 atomes de carbone ou hétéroaryle ayant 2 à 9 atomes de carbone et 1 à 3 hétéroatomes, notamment d'azote, d'oxygène et/ou de soufre, et on considère dans chaque cas comme substituants : un halogène, un radical cyano, nitro, ainsi qu'un radical, à chaîne droite ou ramifiée dans chaque cas, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents, ou bien

$R^5$ et $R^6$ forment conjointement avec l'atome d'azote auquel ils sont liés un hétérocycle pentagonal à décagonal portant éventuellement un ou plusieurs substituants identiques ou différents, qui peut comporter le cas échéant 1 ou 2 autres hétéroatomes, notamment d'azote, d'oxygène et/ou de soufre, et on considère alors comme substituants : un halogène ainsi qu'un groupe, chacun à chaîne droite ou ramifiée, alkyle ou halogénalkyle ayant chacun 1 à 4 atomes de carbone et le cas échéant 1 à 9 atomes d'halogènes identiques ou différents ainsi que 1 ou 2 groupes oxo ou thiono,

$R^7$ désigne un groupe, à chaîne droite ou ramifiée dans chaque cas, alkyle ayant 1 à 8 atomes de carbone, alcényle ayant 2 à 8 atomes de carbone, alcynyle ayant 2 à 8 atomes de carbone, un groupe cycloalkyle ayant 3 à 7 atomes de carbone, un groupe cycloalkylalkyle ayant 3 à 7 atomes de carbone dans la partie cycloalkyle et 1 à 6 atomes de carbone dans la partie alkyle ou un groupe, portant éventuellement dans chaque cas un ou plusieurs substituants identiques ou différents, aralkyle ayant 6 à 10 atomes de carbone dans la partie aryle et 1 à 6 atomes de carbone dans la partie alkyle, ou aryle ayant 6 à 10 atomes de carbone, et on considère alors dans chaque cas comme substituants de la partie aryle : un halogène, un radical cyano, nitro, ainsi qu'un radical, à chaîne droite ou ramifiée dans chaque cas, alkyle, alkoxy ou halogénalkyle ayant chacun 1 à 4 atomes de carbone et le cas échéant 1 à 9 atomes d'halogènes identiques ou différents et

$n$ représente le nombre 0, 1 ou 2,

$R^2$ désigne un groupe, à chaîne droite ou ramifiée dans chaque cas, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, un groupe cycloalkylalkyle ou cycloalkyle ayant chacun 3 à 7 atomes de carbone dans la partie cycloalkyle et le cas échéant 1 à 6 atomes de carbone dans la partie alkyle à chaîne droite ou ramifiée ou un groupe, portant éventuellement dans chaque cas un ou plusieurs substituants identiques ou différents, aralkyle ou aryle ayant chacun 6 à 10 atomes de carbone dans la partie aryle et le cas échéant 1 à 6 atomes de carbone dans la partie alkyle à chaîne droite ou ramifiée, et on considère alors dans chaque cas comme substituants de la partie aryle : un halogène, un radical cyano, nitro, ainsi qu'un

radical, à chaîne droite ou ramifiée dans chaque cas, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents,

$R^3$ est l'hydrogène ou un groupe alkyle à chaîne droite ou ramifiée ayant 1 à 8 atomes de carbone,

$R^4$ est un groupe alkoximinoalkyle à chaîne droite ou ramifiée ayant 1 à 8 atomes de carbone dans chacune des parties alkyle individuelles,

X représente l'oxygène ou le soufre et

Y est l'oxygène ou le soufre,

caractérisé en ce que :

a) on fait réagir des 1-chloro-(thio)-carbonyltriazolinones de formule (II)

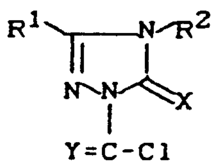

(II)

dans laquelle

$R^1$, $R^2$, X et Y ont la définition indiquée ci-dessus,

avec des amines de formule (III)

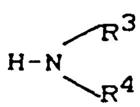

(III)

dans laquelle

$R^3$ et $R^4$ ont la défintion indiquée ci-dessus,

éventuellement en présence d'un diluant et en la présente éventuelle d'un accepteur d'acide, ou bien

b) au cas où $R^3$ désigne l'hydrogène, on fait réagir des triazolinones non substituées en position 1 de formule (IV)

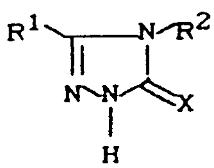

(IV)

dans laquelle

$R^1$, $R^2$ et X ont la définition indiquée ci-dessus, avec des iso-(thio)-cyanates de formule (V),

$R^4$-N = C = Y (V)

dans laquelle

$R^4$ et Y ont la définition indiquée ci-dessous,

le cas échéant en présence d'un diluant et en la présence éventuelle d'une substance auxiliaire de réaction.

**3.** Compositions herbicides, caractérisées par une teneur en au moins une triazolinone substituée de formule (I) suivant les revendications 1 et 2.

**4.** Procédé pour combattre des mauvaises herbes, caractérisé en ce qu'on fait agir des triazolinones substituées de formule (I) suivant les revendications 1 et 2 sur les mauvaises herbes et/ou sur leur

milieu.

5. Utilisation de triazolinones substituées de formule (I) suivant les revendications 1 et 2 pour combattre des mauvaises herbes.

6. Procédé de préparation de compositions herbicides, caractérisé en ce qu'on mélange des triazolinones substituées de formule (I) suivant les revendications 1 et 2 avec des diluants et/ou des substances tensio-actives.